(19) **Europäisches Patentamt / European Patent Office / Office européen des brevets**

(11) **EP 2 415 478 A1**

(12) # EUROPEAN PATENT APPLICATION
published in accordance with Art. 153(4) EPC

(43) Date of publication:
**08.02.2012 Bulletin 2012/06**

(21) Application number: **10758526.7**

(22) Date of filing: **25.03.2010**

(51) Int Cl.:
*A61K 38/00* (2006.01)  *A61P 27/02* (2006.01)
*A61P 43/00* (2006.01)  *C07K 7/06* (2006.01)
*C07K 7/08* (2006.01)  *C07K 14/00* (2006.01)
*C12N 9/99* (2006.01)

(86) International application number:
**PCT/JP2010/055208**

(87) International publication number:
**WO 2010/113753 (07.10.2010 Gazette 2010/40)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **30.03.2009 JP 2009080992**

(71) Applicant: **Santen Pharmaceutical Co., Ltd**
**Osaka-shi**
**Osaka 533-8651 (JP)**

(72) Inventors:
• **HIRAI, Shin-ichiro**
**Ikoma-shi**
**Nara 630-0101 (JP)**

• **MATSUSHITA, Tokiyoshi**
**Ikoma-shi**
**Nara 630-0101 (JP)**
• **FUJITA, Yukie**
**Ikoma-shi**
**Nara 630-0101 (JP)**
• **KURASHIMA, Hiroaki**
**Ikoma-shi**
**Nara 630-0101 (JP)**
• **OOHASHI, Kouji**
**Ikoma-shi**
**Nara 630-0101 (JP)**

(74) Representative: **Müller-Boré & Partner**
**Patentanwälte**
**Grafinger Straße 2**
**81671 München (DE)**

(54) **PROPHYLACTIC OR THERAPEUTIC AGENT FOR RETINAL DISEASES AND METHOD FOR PREVENTING OR TREATING RETINAL DISEASES, EACH COMPRISING JNK (C-JUN N-TERMINAL KINASE)-INHIBITING PEPTIDE, AND USE OF THE PEPTIDE**

(57) Intravitreal administration of a JNK-inhibitory peptide less than 150 amino acids in length, containing at least one D-amino acid, and having (a) a JNK-inhibitory sequence of at least any of SEQ ID NO: 1 and SEQ ID NO: 2, and (b) a transport sequence of at least any of SEQ ID NO: 3 and SEQ ID NO: 4 suppressed spermidine-induced retinal pigment epithelial damage, tunicamycin-induced photoreceptor cell damage, and laser-induced choroidal neovascularization. Thus, the JNK-inhibitory peptide of the present invention is useful for prophylaxis or therapy of a retinal disease. By the use of this JNK-inhibitory peptide, a drug and a method are provided which are capable of preventing or treating a retinal disease even by topical administration to the eye, and use of the JKN-inhibitory peptide for manufacturing the drug is also provided.

**Description**

TECHNICAL FIELD

The present invention relates to a prophylactic or therapeutic agent for a retinal disease containing, as an active ingredient, a JNK-inhibitory peptide including a specific amino acid sequence, less than 150 amino acids in length, and containing at least one D-amino acid. The present invention also relates to a method for prophylaxis or therapy of a retinal disease using such a JNK-inhibitory peptide. Further, the present invention relates to use of such a JNK-inhibitory peptide for manufacturing a prophylactic or therapeutic agent for a retinal disease.

BACKGROUND ART

Retinal diseases are one of the most important family of diseases in the field of ophthalmology. Many retinal diseases are intractable, and serious symptoms that can be a cause of blindness also frequently develop. Representative examples of retinal diseases include age-related macular degeneration (hereinafter also referred to as "AMD"), diabetic retinopathy, central exudative chorioretinopathy, angioid streaks, retinal pigment epithelium detachment, multifocal choroiditis, retinopathy of prematurity, retinitis pigmentosa, Leber's disease, retinal artery occlusion, retinal vein occlusion, central serous chorioretinopathy, retinal macroaneurysm, retinal detachment, proliferative vitreoretinopathy, Stargardt's disease, choroidal sclerosis, chorioderemia, vitelliform macular dystrophy, Oguchi's disease, fundus albipunctatus, retinitis punctata albescens, and gyrate atrophy of choroid and retina. Neovascular maculopathy, caused by high myopia, tilted disc syndrome, choroidal osteoma, or the like, is also known as a retinal disease that affects vision.

Currently, the development of a drug for effectively preventing or treating these retinal diseases is desired. Age-related macular degeneration and diabetic retinopathy, in particular, are major causes of blindness that develops from middle to old age in advanced countries such as Western countries and Japan, and hence, are considered to be very important diseases ophthalmologically and socially.

Age-related macular degeneration, sometimes also regarded as being identical to late age-related maculopathy, is categorized into "atrophic AMD", which causes atrophy in the retinal pigment epithelium and choroidal capillary, and "exudative AMD", in which choroidal neovascular vessels progress at macular region from the choroid, causing hemorrhage and exudative lesions, and finally causing the formation of scar tissue. A peculiar type of exudative age-related macular degeneration, referred to as "polypoidal choroidal vasculopathy", is also known. Polypoidal choroidal vasculopathy causes expansion of choroidal blood vessels under the retina in polypoid form, which causes subretinal hemorrhage, thereby causing the development of pathological conditions similar to those of age-related macular degeneration. Early stages of age-related macular degeneration in which drusen and retinal pigment epithelial abnormalities are observed are particularly referred to as "early age-related maculopathy".

Further, diabetic retinopathy is a retinal blood vessel disorder, which is a diabetic complication. Diabetic retinopathy is generally classified into the following three types: "simple retinopathy", in which retinal macroaneurysm, retinal hemorrhage, retinal edema, and the like are observed; "proliferative retinopathy", which is accompanied by neovascular vessels, vitreous hemorrhage, traction retinal detachment, and the like; and "pre-proliferative retinopathy", which is intermediate between the above two types. Among pathological conditions of diabetic retinopathy, since macular edema caused by an increase in vascular permeability greatly affects vision, a pathological condition of diabetic retinopathy accompanied by macular edema, in particular, is sometimes referred to as "diabetic macular edema".

Retinal diseases other than age-related macular degeneration and diabetic retinopathy will be further described below.

With regard to central exudative chorioretinopathy, angioid streaks, retinal pigment epithelium detachment, and multifocal choroiditis, choroidal neovascularization is considered to be deeply involved in the pathological conditions of all of these diseases, and these diseases are also known as causes of neovascular maculopathy. Similarly, in ocular diseases such as high myopia, tilted disc syndrome, and choroidal osteoma, choroidal neovascularization is sometimes observed, and these diseases may sometimes progress to neovascular maculopathy. It is well known that abnormal vascular proliferation (neovascularization) is an etiology of retinopathy of prematurity. That is, such choroidal neovascularization is believed to be involved in decrease in vision observed in the above-mentioned diseases.

It is known that tetinitis pigmentosa and Leber's disease are diseases in which retinal pigment epithelial cells and photoreceptors are damaged, and that hemeralopia initially develops, followed by a gradual progress of narrowing of the vision field. It is also known that, similarly in retinal artery occlusion, photoreceptors are damaged by ischemia caused by a blocked artery.

Stargardt's disease, choroidal sclerosis, chorioderemia, vitelliform macular dystrophy, Oguchi's disease, fundus albipunctatus, and retinitis punctata albescens are all diseases in which the retinal pigment epithelium is damaged by atrophy, degeneration, or the like, causing decrease in vision. That is, it has been pointed out that degeneration of the retinal pigment epithelium is a cause of Stargardt's disease, and atrophy of the retinal pigment epithelium is one cause of the onset of choroidal sclerosis and chorioderemia. On the other hand, although the pathological conditions of vitelliform macular dystrophy, Oguchi's disease, fundus albipunctatus, and retinitis punctata albescens have not necessarily been clarified, damage to the retinal pigment epithelium is still believed to be one cause of these diseases.

Gyrate atrophy of choroid and retina is an autosomal recessive inheritance disorder caused by a deficiency of metabolic enzyme ornithine. Small circular atrophic lesions appear around the middle of the choroid and retina, and visual disorders such as decrease in vision, hemeralopia, and narrow field vision are observed. Invest. Ophthalmol. Vis. Sci., 48 (1), 455-463 (2007) (NPL 1) discloses that spermidine, which is one of the metabolites of ornithine, damages the retinal pigment epithelial cells, pointing out a correlation between the retinal pigment epithelial damage caused by spermidine and gyrate atrophy of choroid and retina.

In retinal vein occlusion, the permeability of the blood vessel wall is increased by damage to vascular endothelial cells caused by venous occlusion, causing severe retinal edema in the occluded area, leading to neovascularization in a prolonged period (1 to 2 years). Increase in the permeability of choroidal vessels is also considered to be an etiology of central serous chorioretinopathy. Furthermore, it is known that retinal edema is also observed in retinal macroaneurysm.

It is known that retinal detachment is also associated with such ocular diseases as diabetic retinopathy and retinopathy of prematurity mentioned above. A deep correlation is also observed between proliferative vitreoretinopathy and retinosis.

On the other hand, JNKs (c-Jun amino-terminal kinases; c-Jun N-terminal kinases), which are members of a stress-activated group of mitogen-activated protein (MAP) kinases, phosphorylate c-Jun. Japanese National Patent Publication No. 2008-518922 (PTL 1) discloses that intraperitoneal administration of SP600125, which is a non-peptidic JNK inhibitor, protects against ischemia/reperfusion-induced eye neuropathy in rats. PTL 1 also discloses that SP600125 suppresses the death of rat cultured retinal ganglion cells induced by serum deprivation and glutamic acid. On the basis of these findings, PTL 1 describes that JNK inhibitors such as SP600125 can serve as therapeutic agents for ocular diseases such as glaucoma, ischemic neuropathy, ischemic retinopathy, pigmentary retinopathy, and retinal detachment.

Invest. Ophthalmol Vis. Sci., 44 (12), 5383-5395 (2003) (NPL 2), however, indicates that intravitreal administration of SP600125 cannot protect against ischemic retinopathy, but rather tends to aggravate the ischemic disorder. That is, there is a possibility that SP600125 cannot treat or prevent a retinal disease when topically administered to the eye. Generally, the route of administration of a therapeutic agent for a retinal disease is topical administration to the eye, such as intravitreous administration. Topical administration to the eye is preferred also in view of preventing systemic side effects.

Peptidic JNK inhibitors are also known as JNK inhibitors other than SP600125 (NPL 2, Japanese National Patent Publication No. 2003-511071 (PTL 2), Japanese National Patent Publication No. 2009-507502 (PTL 3), and the like). NPL 2, however, indicates that intravitreal administration of a peptidic JNK inhibitor cannot protect against ischemic retinopathy, either, and that it rather tends to aggravate the disorder.

As described above, none of the prior art literatures discloses a JNK inhibitor capable of treating or preventing retinal diseases when topically administered to the eye. Furthermore, no reports exist which indicate that JNK inhibitors other than SP600125 are effective for therapy or prophylaxis of retinal diseases.

CITATION LIST

PATENT LITERATURE

   PTL 1: Japanese National Patent Publication No. 2008-518922
   PTL 2: Japanese National Patent Publication No. 2003-511071
   PTL 3: Japanese National Patent Publication No. 2009-507502

NON PATENT LITERATURE

   NPL 1: Invest. Ophthalmol. Vis. Sci., 48 (1), 455-463 (2007)
   NPL 2: Invest. Ophthalmol. Vis. Sci., 44 (12), 5383-5395 (2003)

SUMMARY OF INVENTION

TECHNICAL PROBLEM

The present invention was made to solve the above-described problems. An object of the invention is to provide a drug and a method including a JNK-inhibitory peptide as an active ingredient and capable of preventing or treating a retinal disease even by topical administration to the eye.

SOLUTION TO PROBLEM

The present inventors conducted extensive research concerning JNK inhibitors capable of treating or preventing retinal diseases, and consequently found that intravitreal administration of a JNK-inhibitory peptide including a specific amino acid sequence, less than 150 amino acids in length, and containing at least one D-amino acid can suppress spermidine-induced retinal pigment epithelial damage, laser-induced choroidal neovascularization, and tunicamycin-induced photoreceptor cell damage, thereby arriving at the present invention. This is a surprising result, considering that similar effects were not confirmed with the representative JNK inhibitor, SP600125. As described in the BACKGROUND ART section, retinal pigment epithelial damage, choroidal neovascularization, and photoreceptor cell damage are deeply involved in the onset and/or progression of many retinal diseases, and hence, a drug for suppressing retinal pigment epithelial damage, choroidal neovascularization, and photoreceptor cell damage is useful in prophylaxis or therapy of a retinal disease.

In summary, the present invention is a prophylactic or therapeutic agent for a retinal disease comprising, as an active ingredient, a JNK-inhibitory peptide less than 150 amino acids in length, containing at least one D-amino acid, and having (a) a JNK-inhibitory sequence of at least any of SEQ ID NO: 1 and SEQ ID NO: 2, and (b) a transport sequence of at least any of SEQ ID NO: 3 and SEQ ID NO: 4.

The present invention also provides a prophylactic or therapeutic agent for a retinal disease comprising, as an active ingredient, a JNK-inhibitory peptide less than 150 amino acids in length, containing at least one D-amino acid, and having an amino acid sequence of at least any of SEQ ID NO: 5 and SEQ ID NO: 6.

Further, the present invention provides a prophylactic or therapeutic agent for a retinal disease comprising, as an active ingredient, a JNK-inhibitory peptide consisting of an amino acid sequence of SEQ ID NO: 5 or SEQ ID NO: 6, and containing at least one D-amino acid. In this case, all of the amino acids in the JNK-inhibitory peptide are preferably D-amino acids.

The retinal disease in the present invention is preferably at least one selected from the group consisting of age-related macular degeneration, diabetic macular edema, diabetic retinopathy (excluding diabetic macular edema), central exudative chorioretinopathy, angioid streaks, retinal pigment epithelium detachment, multifocal choroiditis, neovascular maculopathy (limited only to case caused by high myopia, tilted disc syndrome, or choroidal osteoma), retinopathy of prematurity, retinitis pigmentosa, Leber's disease, retinal artery occlusion, retinal vein occlusion, central serous chorioretinopathy, retinal macroaneurysm, retinal detachment, proliferative vitreoretinopathy, Stargardt's disease, choroidal sclerosis, chorioderemia, vitelliform macular dystrophy, Oguchi's disease, fundus albipunctatus, retinitis punctata albescens, and gyrate atrophy of choroid and retina.

The route of administration of the prophylacic or therapeutic agent for a retinal disease of the present invention is preferably intravitreal administration, administration into the conjunctival sac, subconjunctival administration, or sub-tenon administration.

In the prophylacic or therapeutic agent for a retinal disease of the present invention comprising, as an active ingredient, a JNK-inhibitory peptide consisting of an amino acid sequence of SEQ ID NO: 5 or SEQ ID NO: 6, wherein all of the amino acids are D-amino acids, it is preferred that the retinal disease be at least one selected from the group consisting of age-related macular degeneration, diabetic macular edema, and diabetic retinopathy (excluding diabetic macular edema), and that the route of administration be intravitreal administration.

The present invention also provides a method for prophylaxis or therapy of a retinal disease comprising administering to a patient a pharmacologically effective amount of a JNK-inhibitory peptide less than 150 amino acids in length, containing at least one D-amino acid, and having (a) a JNK-inhibitory sequence of at least any of SEQ ID NO: 1 and SEQ ID NO: 2, and (b) a transport sequence of at least any of SEQ ID NO: 3 and SEQ ID NO: 4.

Further, the present invention provides a method for prophylaxis or therapy of a retinal disease comprising administering to a patient a pharmacologically effective amount of a JNK-inhibitory peptide less than 150 amino acids in length, containing at least one D-amino acid, and having an amino acid sequence of at least any of SEQ ID NO: 5 and SEQ ID NO: 6.

Furthermore, the present invention provides a method for prophylaxis or therapy of a retinal disease comprising administering to a patient a pharmacologically effective amount of a JNK-inhibitory peptide consisting of an amino acid sequence of SEQ ID NO: 5 or SEQ ID NO: 6, and containing at least one D-amino acid.

In the method for prophylaxis or therapy of the present invention, all of the amino acids in the JNK-inhibitory peptide are preferably D-amino acids.

The retinal disease in the present invention is preferably at least one selected from the group consisting of age-related macular degeneration, diabetic macular edema, diabetic retinopathy (excluding diabetic macular edema), central exudative chorioretinopathy, angioid streaks, retinal pigment epithelium detachment, multifocal choroiditis, neovascular maculopathy (limited only to case caused by high myopia, tilted disc syndrome, or choroidal osteoma), retinopathy of prematurity, retinitis pigmentosa, Leber's disease, retinal artery occlusion, retinal vein occlusion, central serous chorioretinopathy, retinal macroaneurysm, retinal detachment, proliferative vitreoretinopathy, Stargardt's disease, choroidal sclerosis, chorioderemia, vitelliform macular dystrophy, Oguchi's disease, fundus albipunctatus, retinitis punctata albescens, and gyrate atrophy of choroid and retina.

The route of administration in the method for prophylaxis or therapy of a retinal disease of the present invention is preferably intravitreal administration, administration into the conjunctival sac, subconjunctival administration, or sub-tenon administration.

In the method for prophylaxis or therapy of a retinal disease of the present invention in the case of administering to a patient a pharmacologically effective amount of a JNK-inhibitory peptide consisting of an amino acid sequence of SEQ ID NO: 5 or SEQ ID NO: 6, wherein all of the amino acids are D-amino acids, it is preferred that the retinal disease be at least one selected from the group consisting of age-related macular degeneration, diabetic macular edema, and diabetic retinopathy (excluding diabetic macular edema), and that the route of administration be intravitreal administration.

The present invention also provides use of a JNK-inhibitory peptide for manufacturing a prophylactic or therapeutic agent for a retinal disease, the JNK-inhibitory peptide being less than 150 amino acids in length, containing at least one D-amino acid, and having (a) a JNK-inhibitory sequence of at least any of SEQ ID NO: 1 and SEQ ID NO: 2, and (b) a transport sequence of at least any of SEQ ID NO: 3 and SEQ ID NO: 4.

Moreover, the present invention provides use of a JNK-inhibitory peptide for manufacturing a prophylactic or therapeutic agent for a retinal disease, the JNK-inhibitory peptide being less than 150 amino acids in length, containing at least one D-amino acid, and having an amino acid sequence of at least any of SEQ ID NO: 5 and SEQ ID NO: 6.

Further, the present invention provides use of a JNK-inhibitory peptide for manufacturing a prophylactic or therapeutic agent for a retinal disease, the JNK-inhibitory peptide consisting of an amino acid sequence of SEQ ID NO: 5 or SEQ ID NO: 6, and containing at least one D-amino acid.

In the use of the JNK-inhibitory peptide of the present invention, all of the amino acids in the JNK-inhibitory peptide are preferably D-amino acids.

The retinal disease in the present invention is preferably at least one selected from the group consisting of age-related macular degeneration, diabetic macular edema, diabetic retinopathy (excluding diabetic macular edema), central exudative chorioretinopathy, angioid streaks, retinal pigment epithelium detachment, multifocal choroiditis, neovascular maculopathy (limited only to case caused by high myopia, tilted disc syndrome, or choroidal osteoma), retinopathy of prematurity, retinitis pigmentosa, Leber's disease, retinal artery occlusion, retinal vein occlusion, central serous chorioretinopathy, retinal macroaneurysm, retinal detachment, proliferative vitreoretinopathy, Stargardt's disease, choroidal sclerosis, chorioderemia, vitelliform macular dystrophy, Oguchi's disease, fundus albipunctatus, retinitis punctata albescens, and gyrate atrophy of choroid and retina.

The route of administration in the use of the JNK-inhibitory peptide of the present invention is preferably intravitreal administration, administration into the conjunctival sac, subconjunctival administration, or sub-tenon administration.

In the use of the JNK-inhibitory peptide consisting of an amino acid sequence of SEQ ID NO: 5 or SEQ ID NO: 6, wherein all of the amino acids are D-amino acids, for manufacturing a prophylactic or therapeutic agent for a retinal disease, it is preferred that the retinal disease be at least one selected from the group consisting of age-related macular degeneration, diabetic macular edema, and diabetic retinopathy (excluding diabetic macular edema), and that the route of administration be intravitreal administration.

ADVANTAGEOUS EFFECTS OF INVENTION

As will be described later, intravitreal administration of a JNK-inhibitory peptide including a specific amino acid sequence wherein at least one of the amino acids is a D-amino acid suppressed spermidine-induced retinal pigment epithelial damage, laser-induced choroidal neovascularization, and tunicamycin-induced photoreceptor cell damage. That is, the JNK-inhibitory peptide has a surprising effect of suppressing all of retinal pigment epithelial damage, photoreceptor cell damage, and choroidal neovascularization, which are etiologies of many retinal diseases. Therefore, by incorporating the JNK-inhibitory peptide as an active ingredient, a drug and a method capable of preventing or treating a retinal disease even by topical administration to the eye can be provided, and use of the JNK-inhibitory peptide for manufacturing the above-described drug is also provided.

DESCRIPTION OF EMBODIMENTS

The present invention provides a prophylactic or therapeutic agent for a retinal disease comprising, as an active ingredient, a JNK-inhibitory peptide less than 150 amino acids in length, containing at least one D-amino acid, and having (a) a JNK-inhibitory sequence of at least any of SEQ ID NO: 1 and SEQ ID NO: 2, and (b) a transport sequence of at least any of SEQ ID NO: 3 and SEQ ID NO: 4. The present invention also provides a method for prophylaxis or therapy of a retinal disease comprising administering a pharmacologically effective amount of such a JNK-inhibitory peptide to a patient. The present invention further provides use of such a JNK-inhibitory peptide for manufacturing a prophylactic or therapeutic agent for a retinal disease.

As used herein, the term "JNK-inhibitory peptide" means a peptide having activity that suppresses the phosphorylation of a substrate (such as c-Jun) by JNK (hereinafter also referred to as "JNK-inhibitory activity"). The JNK-inhibitory activity can be readily measured using a commercially available JNK activity assay kit (manufactured by Cell Signaling Technology, SAPK/JNK Assay Kit (Cat. No. 9810), etc.), and can also be measured according to the methods disclosed in Japanese National Patent Publication No. 2003-511071 (PTL 2) and Japanese National Patent Publication No. 2009-507502 (PTL 3).

As used herein, the phrase "a JNK-inhibitory sequence of at least any of SEQ ID NO: 1 and SEQ ID NO: 2" means either one or both of the amino acid sequences $NH_2$-RPKRPTTLNLFPQVPRSQD-COOH (SEQ ID NO: 1) and $NH_2$-DQSRPVQPFLNLTTPRKPR-COOH (SEQ ID NO: 2), which bind with JNK and inhibit its activity. Here, the JNK-inhibitory sequence can be composed of L-amino acids, D-amino acids, or a combination of both. Preferably, however, at least one of the amino acids in the JNK-inhibitory sequence is a D-amino acid, and more preferably, all of the amino acids in the JNK-inhibitory sequence are D-amino acids.

As used herein, the phrase "a transport sequence of SEQ ID NO: 3 and SEQ ID NO: 4" means either one or both of the amino acid sequences $NH_2$-GRKKRRQRRR-COOH (SEQ ID NO: 3) and $NH_2$-RRRQRRKKRG-COOH (SEQ ID NO: 4), which induce the peptide in desired cells. Here, the transport sequence can be composed of L-amino acids, D-amino acids, or a combination of both. Preferably, however, at least one of the amino acids in the transport sequence is a D-amino acid, and more preferably, all of the amino acids in the transport sequence are D-amino acids.

Preferably, the JNK-inhibitory peptide of the present invention has at least any of the amino acid sequences $NH_2$-GRKKRRQRRRPPRPKRPTTLNLFPQVPRSQD-COOH (SEQ ID NO: 5) and $NH_2$-GRKKRRQRRRPPRPKRPT-TLNLFPQVPRSQDT-COOH (SEQ ID NO: 6), less than 150 amino acids in length, and contains at least one D-amino acid.

Moreover, preferably, the JNK-inhibitory peptide of the present invention consists of the amino acid sequence $NH_2$-GRKKRRQRRRPPRPKRPTTLNLFPQVPRSQD-COOH (SEQ ID NO: 5) or $NH_2$-GRKKRRQRRRPPRPKRPT-TLNLFPQVPRSQDT-COOH (SEQ ID NO: 6), wherein at least one of the amino acids is a D-amino acid.

Furthermore, in the JNK-inhibitory peptide of the present invention, all of the amino acids are particularly preferably D-amino acids.

One specific example of the most preferred JNK-inhibitory peptide of the present invention is a peptide consisting of the amino acid sequence $NH_2$-GRKKRRQRRRPPRPKRPTTLNLFPQVPRSQD-COOH (SEQ ID NO: 5), wherein all of the amino acids are D-amino acids (hereinafter also referred to as a "peptide A").

Here, the sequences of SEQ ID NOs: 1 to 6 are shown in Table 1.

[Table 1]

| SEQ ID NO: | Number of Amino Acids | Amino Acid Sequence |
|---|---|---|
| 1 | 19 | RPKRPTTLNL FPQVPRSQD ($NH_2$-RPKRPTTLNLFPQVPRSQD-COOH) |
| 2 | 19 | DQSRPVQPFL NLTTPRKPR ($NH_2$-DQSRPVQPFLNLTTPRKPR-COOH) |
| 3 | 10 | GRKKRRQRRR ($NH_2$-GRKKRRQRRR-COOH) |
| 4 | 10 | RRRQRRKKRG ($NH_2$-RRRQRRKKRG-COOH) |
| 5 | 31 | GRKKRRQRRR PPRPKRPTTL NLFPQVPRSQ D ($NH_2$-GRKKRRQRRRPPRPKRPTTLNLFPQVPRSQD-COOH) |
| 6 | 32 | GRKKRRQRRR PPRPKRPTTL NLFPQVPRSQ DT ($NH_2$-GRKKRRQRRRPPRPKRPTTLNLFPQVPRSQDT-COOH) |

The JNK-inhibitory peptide of the present invention can be synthesized according to chemical synthesis on a solid phase using a commercially available peptide synthesizing apparatus, or can also be synthesized according to the method disclosed in Japanese National Patent Publication No. 2009-507502 (PTL 3). Peptide A is marketed under the trade name "D-JNKiI" (Cat. No. EI-355) by BIOMOL.

Examples of retinal diseases in the present invention include age-related macular degeneration, diabetic macular edema, diabetic retinopathy (excluding diabetic macular edema), central exudative chorioretinopathy, angioid streaks, retinal pigment epithelium detachment, multifocal choroiditis, retinopathy of prematurity, retinitis pigmentosa, Leber's disease, retinal artery occlusion, retinal vein occlusion, central serous chorioretinopathy, retinal macroaneurysm, retinal detachment, proliferative vitreoretinopathy, Stargardt's disease, choroidal sclerosis, chorioderemia, vitelliform macular dystrophy, Oguchi's disease, fundus albipunctatus, retinitis punctata albescens, and gyrate atrophy of choroid and retina. Neovascular maculopathy caused by high myopia, tilted disc syndrome, and choroidal osteoma is also included in the retinal diseases in the present invention. The prophylactic or therapeutic agent for a retinal disease of the present invention can be suitably applied to the prophylaxis or therapy of at least one of these retinal diseases.

As described above, in the present invention, early age-related maculopathy, atrophic AMD, and exudative AMD are included in age-related macular degeneration, and polypoidal choroidal vasculopathy is also included in age-related macular degeneration. Simple diabetic retinopathy, pre-proliferative diabetic retinopathy, and proliferative diabetic retinopathy are included in diabetic retinopathy (excluding diabetic macular edema).

Central retinal artery occlusion and branch retinal artery occlusion are included in retinal artery occlusion. Central retinal vein occlusion and branch retinal vein occlusion are also included in retinal vein occlusion.

Among the above-mentioned retinal diseases, the JNK-inhibitory peptide of the present invention is particularly effective for age-related macular degeneration, diabetic macular edema, and diabetic retinopathy (excluding diabetic macular edema).

The JNK-inhibitory peptide of the present invention can optionally be mixed with pharmaceutically acceptable additives and formulated as a single preparation or a combination preparation by using a widely used technique.

When the JNK-inhibitory peptide of the present invention is used for prophylaxis or therapy of the above-mentioned retinal diseases, it can be orally or parenterally administered to a patient. Examples of forms of administration include oral administration, intravenous administration, topical administration to the eye (for example, instillation, administration into the conjunctival sac, intravitreal administration, subconjunctival administration, and sub-tenon administration), and dermal administration. Among the above, intravitreal administration, administration into the conjunctival sac, subconjunctival administration, or sub-tenon administration is preferred, and intravitreal administration is particularly preferred.

Particularly preferably, the prophylactic or therapeutic agent for a retinal disease of the present invention contains, as an active ingredient, a JNK-inhibitory peptide consisting of the amino acid sequence of SEQ ID NO: 5 or SEQ ID NO: 6, wherein all of the amino acids are D-amino acids, in which case the retinal disease is at least one selected from the group consisting of age-related macular degeneration, diabetic macular edema, and diabetic retinopathy (excluding diabetic macular edema), and the route of administration is intravitreal administration. This also applies to the method for prophylaxis or therapy of a retinal disease and use of the JNK-inhibitory peptide of the present invention.

The JNK-inhibitory peptide of the present invention is optionally formulated into dosage forms suitable for administration, together with pharmaceutically acceptable additives. Examples of dosage forms suitable for oral administration include tablets, capsules, granules, fine granules, and powders; and dosage forms suitable for parenteral administration include injections, eye drops, ophthalmic ointments, patches, gels, and intercalating agents. These dosage forms can be prepared using general techniques widely used in the art. In addition to these preparations, the JNK-inhibitory peptide of the present invention can also be formulated into preparations for intraocular implants, or formulations made into DDS (Drug Delivery Systems), such as microspheres.

For example, tablets can be prepared by using an additive selected, as appropriate, from, for example, excipients such as lactose, glucose, D-mannitol, dicalcium phosphate anhydrous, starch, and sucrose; disintegrating agents such as carboxymethyl cellulose, carboxymethyl cellulose calcium, croscarmellose sodium, crospovidone, starch, partially pregelatinized starch, and low-substituted hydroxypropylcellulose; binders such as hydroxypropylcellulose, ethyl cellulose, gum arabic, starch, partially pregelatinized starch, polyvinylpyrrolidone, and polyvinyl alcohol; lubricants such as magnesium stearate, calcium stearate, talc, hydrous silica dioxide, and hydrogenated oil; coating agents such as purified sucrose, hydroxypropylmethyl cellulose, hydroxypropyl cellulose, methyl cellulose, ethyl cellulose, and polyvinylpyrrolidone; and flavoring agents such as citric acid, aspartame, ascorbic acid, and menthol.

Injections can be prepared by optionally using an additive selected from, for example, isotonizing agents such as sodium chloride; buffering agents such as sodium phosphate; surfactants such as polyoxyethylene sorbitan monooleate; and thickeners such as methyl cellulose.

Eye drops can be prepared by optionally using an additive selected from, for example, isotonizing agents such as sodium chloride and concentrated glycerin; buffering agents such as sodium phosphate and sodium acetate; surfactants such as polyoxyethylene sorbitan monooleate, polyoxyl 40 stearate, and polyoxyethylene hydrogenated castor oil; stabilizers such as sodium citrate and sodium edetate; and preservatives such as benzalkonium chloride and paraben. The pH of the eye drops may be within an ophthalmologically acceptable range, but is normally preferably in the range from 4 to 8. Ophthalmic ointments can be prepared by using widely used bases such as white petrolatum and liquid paraffin.

Intercalating agents can be prepared by grinding and mixing biodegradable polymers, for example, hydroxypropyl cellulose, hydroxypropylmethyl cellulose, carboxyvinyl polymer, and polyacrylic acid, together with the present compound, followed by compression-molding the resulting powders. Excipients, binders, stabilizers, and pH adjusters can be optionally used. Preparations for intraocular implants can be prepared by using biodegradable polymers, for example, polylactic acid, polyglycolic acid, lactic acid-glycolic acid copolymer, and hydroxypropyl cellulose.

The amount of the JNK-inhibitory peptide of the present invention administered may be varied, as appropriate, depending on the dosage form, the severity of the symptoms, the age, and the body weight of the patient to whom the peptide is to be administered, the doctor's judgment, etc. However, in the case of oral administration, 0.01 to 5000 mg, preferably 0.1 to 2500 mg, and more preferably 1 to 1000 mg per day of the JNK-inhibitory peptide may be generally administered to an adult in a single dose or divided doses. In the case of intravenous administration, 0.01 to 5000 mg, preferably 0.1 to 2500 mg, and more preferably 1 to 1000 mg per day of the JNK-inhibitory peptide may be administered to an adult in a single dose or divided doses.

In the case of topical administration to the eye (excluding instillation), 0.00001 to 10 mg, preferably 0.00005 to 5 mg, and more preferably 0.0001 to 1 mg per day of the JNK-inhibitory peptide may be administered to an adult in a single dose or divided doses. In the case of eye drops or an intercalating agent, eye drops or an intercalating agent containing the active ingredient in a concentration of 0.000001 to 10% (w/v), preferably 0.00001 to 1% (w/v), and more preferably 0.0001 to 0.1 % (w/v) may be administered in a single dose or divided doses a day. In the case of a patch, a patch having a content of 0.00001 to 1000 mg may be applied to an adult; and in the case of a preparation for intraocular implants, a preparation for intraocular implants having a content of 0.00001 to 1000 mg may be implanted into the eye of an adult.

While results of pharmacological tests and preparation examples will be demonstrated below, these examples are intended to provide better understanding of the present invention, and in no way limit the scope of the present invention.

[Examples]

[Pharmacological Test 1]

As described in the BACKGROUND ART section, it has been revealed that spermidine induces retinal pigment epithelial damage, consequently leading to photoreceptor cell damage. Therefore, a spermidine-induced retinal degeneration model is used as a model for evaluating therapeutic agents for diseases involving retinal pigment epithelial damage, such as atrophic AMD (Invest. Ophthalmol. Vis. Sci., 48 (1), 455-463 (2007) (NPL 1); Invest. Ophthalmol. Vis. Sci. 51, ARVO E-abstract 3644 (2010); etc.). Thus, an effect of the JNK-inhibitory peptide of the present invention on photoreceptor cell damage caused by retinal pigment epithelial damage was investigated using the aforementioned model, and then compared with that obtained by the representative JNK-inhibitor, SP600125.

(Preparation of Spermidine-Induced Retinal Degeneration Model and Drug Administration)

General anesthesia was induced in rats by intramuscular administration of 1 mL/kg of a mixture (7:1) of a 5% (W/V) ketamine hydrochloride injection solution and a 2% xylazine hydrochloride injection solution. A 0.5% (W/V) tropicamide-0.5% phenylephrine hydrochloride ophthalmic solution was instilled into their eyes to cause dilation of the pupils. Under conditions that ocular fundus is visualized, a microsyringe (25 μL volume, Hamilton) fitted with a 33G needle was used and inserted into the vitreous cavity. To a control group, 10 μL of Dulbecco PBS was administered. To a vehicle-administered group, 10 μL of a 2 mM solution of spermidine dissolved in Dulbecco PBS was administered. To a peptide A-administered group, a 4 mM solution of spermidine dissolved in Dulbecco PBS was mixed with 0.6 mg/mL peptide A dissolved in Dulbecco PBS to give a 1:1 mixture, and 10 μL of the solution was administered into the vitreous cavity. To an SP600125-administered group, spermidine was dissolved in a 0.3 mg/mL solution of SP600125 dissolved in Dulbecco PBS to give a concentration of 2 mM, and 10 μL of the solution was administered into the vitreous cavity. As spermidine, "Spermidine, Trihydrochloride" (Cat. No. 56766), purchased from Calbiochem, was used. As peptide A, "D-JNKiI' " (Cat. No. EI-355), purchased from BIOMOL, was used. As SP600125, "SP600125" (Cat. No. S5567-50MG), purchased from Sigma Aldrich, was used.

(Evaluation)

After 13 days from the administration of spermidine, general anesthesia was induced in the rats under dark adaptation by intramuscular administration of 1 mL/kg of a mixture (7:1) of a 5% (W/V) ketamine hydrochloride injection solution and a 2% xylazine hydrochloride injection solution. A 0.5% (W/V) tropicamide-0.5% phenylephrine hydrochloride ophthalmic solution was instilled into their eyes to cause dilation of the pupils. Then, using an electroretinogram (ERG) measurement apparatus (manufactured by Tomey Corporation, portable ERG&VEP LE-3000), ERGs (a-wave) were measured. Suppression ratios against reduction in ERG amplitude were subsequently calculated using an equation 1 shown below. The results are shown in Table 2.

[Equation 1]

Suppression ratio against reduction in ERG amplitude (%) = $((E_x - E_v)/(E_c - E_v)) \times 100$,

where:

$E_c$: an amplitude value of the a-wave for the control group;
$E_v$: an amplitude value of the a-wave for the vehicle-administered group; and
$E_x$: an amplitude value of the a-wave for the drug-administered group.

[Table 2]

| Amount of Drug Administered | Suppression ratio against Reduction in ERG (a-Wave) amplitude (%) |
| --- | --- |
| Peptide A 3 $\mu$g/eye | 72.7% |
| SP600125 3 $\mu$g/eye | - 17.6% |

(Results)

As shown in Table 2, intravitreal administration of peptide A suppressed the reduction in ERG amplitude by approximately 73% in the spermidine-induced retinal degeneration model. On the other hand, intravitreal administration of SP600125 aggravated the reduction in ERG amplitude by approximately 18%.

(Discussion)

The foregoing results suggested that the JNK-inhibitory peptides of the present invention, including peptide A, suppress spermidine-induced retinal pigment epithelial damage and the resulting photoreceptor cell damage. As described in the BACKGROUND ART section, retinal pigment epithelial damage and the resulting photoreceptor cell damage are believed to be involved in the onset and/or progression of such retinal diseases as age-related macular degeneration (in particular, early age-related maculopathy and atrophic AMD), retinitis pigmentosa, Leber's disease, Stargardt's disease, choroidal sclerosis, chorioderemia, vitelliform macular dystrophy, Oguchi's disease, fundus albipunctatus, retinitis punctata albescens, and gyrate atrophy of choroid and retina. Therefore, the JNK-inhibitory peptide of the present invention is believed to be useful for prophylaxis or therapy of retinal diseases including age-related macular degeneration (in particular, early age-related maculopathy and atrophic AMD).
On the other hand, the representative JNK-inhibitor, SP600125, demonstrated no damage suppression effect in the present model, and the possibility was indicated that SP600125 rather aggravates retinal damage, as described in the BACKGROUND ART section. It is surprising that the JNK-inhibitory peptide of the present invention possessing JNK-inhibitory activity has a retinal pigment epithelial damage suppression effect and a photoreceptor cell damage suppression effect, which are not observed in the representative JNK inhibitor.

[Pharmacological Test 2]

As a pharmacological test model for investigating an inhibitory effect on choroidal neovascularization, a laser-induced choroidal neovascularization model is widely used (Folia Ophthalmologica Japonica, 45, 853-856 (1994)). This model was thus used to evaluate the usefulness of the JNK-inhibitory peptide of the present invention.

(Preparation of Laser-Induced Rat Choroidal Neovascularization Model)

General anesthesia was induced in rats by intramuscular administration of I mL/kg of a mixture (7: 1) of a 5% (W/V) ketamine hydrochloride injection solution and a 2% xylazine hydrochloride injection solution. A 0.5% (W/V) tropicamide-0.5% phenylephrine hydrochloride ophthalmic solution was instilled into their eyes to cause dilation of the pupils. Photocoagulation was subsequently performed using a krypton laser photocoagulator (manufactured by NIDEK, a multicolor laser photocoagulator MC-7000). With focusing laser beam on the deep retinal layers, photocoagulation was conducted in the posterior portion of the ocular fundus at 8 spots per eye in a dispersed manner, while avoiding thick retinal blood vessels. After photocoagulation, fundus photography was performed to identify the laser-irradiated sites.

(Drug Administration)

Immediately after the preparation of the model, under conditions that ocular fundus is visualized, a microsyringe (25 μL volume, Hamilton) fitted with a 33G needle was used and inserted into the vitreous cavity, and 5 μL of a solution to be administered, containing 0.6 mg/mL peptide A dissolved using physiological saline, was administered (a drug-administered group). As peptide A, "D-JNKil" (Cat. No. EI-355), purchased from BIOMOL, was used. To a vehicle-administered group, 5 μL of physiological saline was administered.

(Evaluation)

On day 7 after photocoagulation, general anesthesia was induced in the rats by intramuscular administration of 1 mL/kg of a mixture (7: 1) of a 5% (W/V) ketamine hydrochloride injection solution and a 2% xylazine hydrochloride injection solution. A 0.5% (W/V) tropicamide-0.5% phenylephrine hydrochloride ophthalmic solution was instilled into their eyes to cause dilation of the pupils. Subsequently, 0.1 mL of a 10% fluorescein sodium solution was injected via the tail vein, and fluorescent fundus angiography (manufactured by Kowa, Fundus Camera Kowa PROIII) was performed. In fluorescent fundus angiography, spots at which fluorescence exposure was not observed was determined as negative (without neovascularization), and spots at which fluorescence exposure was observed were determined as positive (with neovascularization). Moreover, when there were two photocoagulation sites at which slight fluorescence exposure was observed, these photocoagulation sites were determined as positive (with neovascularization). Next, a choroidal neovascularization development ratio (%) was calculated from the number of positive spots relative to the 8 laser-irradiated spots, in accordance with an equation 2 shown below, and a suppression ratio (%) of the drug to be evaluated was calculated in accordance with an equation 3 shown below. The results are shown in Table 3. The number of cases in each of the drug-administered group and the vehicle-administered group was 8.

[Equation 2]

$$\text{Choroidal neovascularization generation ratio (\%)} = (\text{the number of positive spots/the total number of photocoagulation sites}) \times 100$$

[Equation 3]

$$\text{Suppression ratio (\%)} = ((A_0 - A_X)/A_0) \times 100,$$

where:

$A_0$: a choroidal neovascularization development ratio of the vehicle-administered group; and
$A_x$: a choroidal neovascularization development ratio of the drug-administered group.

[Table 3]

| Amount of Drug Administered | Suppression Ratio (%) of Choroidal Neovascularization Development |
| --- | --- |
| Peptide A 3 μg/eye | 14.3 |

(Results)

As shown in Table 3, intravitreal administration of peptide A suppressed choroidal neovascularization in the laser-induced rat choroidal neovascularization model by approximately 14%.

(Discussion)

As described in the BACKGROUND ART section, choroidal neovascularization is a principal lesion found in exudative AMD. Neovascularization is also known as one of the major findings in diabetic retinopathy (in particular, proliferative diabetic retinopathy). Further, choroidal neovascularization is also believed to be involved in the onset and/or progression of such retinal diseases as central exudative chorioretinopathy, angioid streaks, retinal pigment epithelium detachment, multifocal choroiditis, neovascular maculopathy (limited only to case caused by high myopia, tilted disc syndrome, or choroidal osteoma), and retinopathy of prematurity. Therefore, the JNK-inhibitory peptide of the present invention is believed to be useful for prophylaxis or therapy of retinal diseases including age-related macular degeneration (in particular, exudative AMD) and diabetic retinopathy (in particular, proliferative retinopathy).

[Pharmacological Test 3]

It has been suggested that endoplasmic reticulum stress is involved in retinal diseases such as age-related macular degeneration and retinitis pigmentosa (Expert Rev. Ophthalmol. 3 (1), 29-42 (2008)). It has also been reported that the administration of tunicamycin, which is an endoplasmic reticulum stress inducer, into the vitreous body induces damage to photoreceptor cells (Nature 311, 575-577 (1984)). Such a model was thus used to evaluate the usefulness of the JNK-inhibitory peptide of the present invention against photoreceptor cell damage.

(Preparation of Tunicamycin-Induced Rat Photoreceptor Cell Damage Model and Drug Administration)

General anesthesia was induced in rats by intramuscular administration of 1 mL/kg of a mixture (7:1) of a 5% (W/V) ketamine hydrochloride injection solution and a 2% xylazine hydrochloride injection solution. A 0.5% (W/V) tropicamide-0.5% phenylephrine hydrochloride ophthalmic solution was instilled into their eyes to cause dilation of the pupils. Under conditions that ocular fundus is visualized, a microsyringe (25 $\mu$L volume, Hamilton) fitted with a 33G needle was used and inserted into the vitreous cavity. To a control group, 5 $\mu$L of a mixture (1:9) of dimethyl sulfoxide and physiological saline was administered. To a vehicle-administered group, 5 $\mu$L of a mixture (1:9) of a 200 $\mu$g/mL solution of tunicamycin dissolved in dimethyl sulfoxide and physiological saline was administered. To a peptide A-administered group, 5 $\mu$L of a mixture (1:9) of a 200 $\mu$g/mL solution of tunicamycin dissolved in dimethyl sulfoxide and 0.6 mg/mL peptide A dissolved in physiological saline was administered into the vitreous cavity. As tunicamycin, "tunicamycin derived from streptomyces SP" (Cat. No. T7765), purchased from Sigma Aldrich, was used. As peptide A, "D-JNKil" (Cat. No. EI-355), purchased from BIOMOL, was used.

(Evaluation)

After 7 days from the administration of tunicamycin, general anesthesia was induced in the rats under dark adaptation by intramuscular administration of 1 mL/kg of a mixture (7:1) of a 5% (W/V) ketamine hydrochloride injection solution and a 2% xylazine hydrochloride injection solution. A 0.5% (WN) tropicamide-0.5% phenylephrine hydrochloride ophthalmic solution was instilled into their eyes to cause dilation of the pupils. Then, using an electroretinogram (ERG) measurement apparatus (manufactured by Tomey Corporation, portable ERG&VEP LE-3000), ERGs (a-wave) were measured. Suppression ratio against reduction in ERG amplitude was subsequently calculated using an equation 4 shown below. The results are shown in Table 4.

[Equation 4]

$$\text{Suppression ratio against reduction in ERG amplitude}(\%) = ((B_x - B_v)/(B_c - B_v)) \times 100,$$

where:

$B_c$: an amplitude value of the a-wave for the control group;

$B_v$: an amplitude value of the a-wave for the vehicle-administered group; and

$B_x$: an amplitude value of the a-wave for the drug-administered group.

[Table 4]

| Amount of Drug Administered | Suppression Ratio against Redaction in ERG (a-Wave) Amplitude (%) |
|---|---|
| Peptide A 2.7 μg/eye | 50.0 |

(Results)

As shown in Table 4, peptide A suppressed the reduction in ERG amplitude by approximately 50% in the tunicamycin-induced photoreceptor cell damage model.

(Discussion)

As described above, endoplasmic reticulum stress is deeply involved in the pathological conditions of such retinal diseases as age-related macular degeneration (in particular, early age-related maculopathy and atrophic AMD) and retinitis pigmentosa, and damage to photoreceptor cells is a principal cause of decrease in vision. It is also known that damage to photoreceptor cells is observed in such retinal diseases as Leber's disease and retinal artery occlusion. Therefore, the JNK-inhibitory peptide of the present invention is believed to be useful for prophylaxis or therapy of retinal diseases including age-related macular degeneration (in particular, early age-related maculopathy and atrophic AMD).

[Preparation Examples]

Drugs of the present invention will be more specifically described with reference to preparation examples; however, the present invention is not limited only thereto.

(Formulation Example 1: Injection)

In 10 ml:

| | |
|---|---|
| Peptide A | 10 mg |
| Sodium chloride | 90 mg |
| Polysorbate 80 | q. s. |
| Sterile purified water | q. s. |

Peptide A and the other components listed above are dissolved in sterile purified water to prepare an injection. By varying the amount of peptide A added, an injection containing 0.1 mg, 1 mg, or 50 mg of peptide A in 10 ml can be prepared.

(Formulation Example 2: Eye Drops (0.01% (w/v)))

In 100 ml:

| | |
|---|---|
| Peptide A | 10 mg |
| Sodium chloride | 900 mg |
| Polysorbate 80 | q. s. |
| Disodium hydrogen phosphate | q. s. |
| Sodium dihydrogen phosphate | q. s. |
| Sterile purified water | q. s. |

Peptide A and the other components listed above are added to sterile purified water and thoroughly mixed to prepare an ophthalmic solution. By varying the amount of peptide A added, eye drops containing peptide A in a concentration of 0.05% (w/v), 0.1 % (w/v), 0.5% (w/v), or 1% (w/v) can be prepared.

(Formulation Example 3: Tablets)

In 100 mg:

| | |
|---|---|
| Peptide A | 1 mg |
| Lactose | 66.4 mg |
| Corn starch | 20 mg |
| Carboxymethyl cellulose calcium | 6 mg |
| Hydroxypropyl cellulose | 6 mg |
| Magnesium stearate | 0.6 mg |

Peptide A and lactose are mixed in a mixer. Carboxymethyl cellulose calcium and hydroxypropyl cellulose are added to the mixture, and the mixture is granulated. The resulting granules are dried and then subjected to particle size regulation, magnesium stearate is added to the particle-size-regulated granules and mixed, after which the mixture is tableted in a tableting machine. By varying the amount of peptide A added, tablets containing 0.1 mg, 10 mg, or 50 mg of peptide A in 100 mg can be prepared.

INDUSTRIAL APPLICABILITY

Intravitreal administration of the JNK-inhibitory peptide of the present invention suppressed spermidine-induced retinal pigment epithelial damage, tunicamycin-induced photoreceptor cell damage, and laser-induced choroidal neovascularization. That is, the JNK-inhibitory peptide of the present invention has a surprising effect of suppressing all of retinal pigment epithelial damage, photoreceptor cell damage, and choroidal neovascularization, which are etiologies of many retinal diseases.
Hence, the JNK-inhibitory peptide of the present invention is useful as a prophylactic or therapeutic agent for a retinal disease and in a method for prophylaxis or therapy of a retinal disease.

**Claims**

1. A prophylactic or therapeutic agent for a retinal disease comprising, as an active ingredient, a JNK (c-Jun amino-terminal kinase)-inhibitory peptide less than 150 amino acids in length, containing at least one D-amino acid, and having (a) a JNK - inhibitory sequence of at least any of SEQ ID NO: 1 and SEQ ID NO: 2, and (b) a transport sequence of at least any of SEQ ID NO: 3 and SEQ ID NO: 4.

2. A prophylactic or therapeutic agent for a retinal disease comprising, as an active ingredient, a JNK-inhibitory peptide less than 150 amino acids in length, containing at least one D-amino acid, and having an amino acid sequence of at least any of SEQ ID NO: 5 and SEQ ID NO: 6.

3. A prophylactic or therapeutic agent for a retinal disease comprising, as an active ingredient, a JNK-inhibitory peptide consisting of an amino acid sequence of SEQ ID NO: 5 or SEQ ID NO: 6, and containing at least one D-amino acid.

4. The prophylactic or therapeutic agent according to claim 3, wherein
all of the amino acids in said JNK-inhibitory peptide are D-amino acids.

5. The prophylactic or therapeutic agent according to any of claims 1 to 4, wherein
said retinal disease is at least one selected from the group consisting of age-related macular degeneration, diabetic macular edema, diabetic retinopathy (excluding diabetic macular edema), central exudative chorioretinopathy, angioid streaks, retinal pigment epithelium detachment, multifocal choroiditis, neovascular maculopathy (limited only to case caused by high myopia, tilted disc syndrome, or choroidal osteoma), retinopathy of prematurity, retinitis pigmentosa, Leber's disease, retinal artery occlusion, retinal vein occlusion, central serous chorioretinopathy, retinal macroaneurysm, retinal detachment, proliferative vitreoretinopathy, Stargardt's disease, choroidal sclerosis, chorioderemia, vitelliform macular dystrophy, Oguchi's disease, fundus albipunctatus, retinitis punctata albescens, and gyrate atrophy of choroid and retina.

6. The prophylactic or therapeutic agent according to any of claims 1 to 4, wherein
a route of administration is intravitreal administration, administration into conjunctival sac, subconjunctival administration, or sub-tenon administration.

7. The prophylactic or therapeutic agent according to claim 4, wherein
said retinal disease is at least one selected from the group consisting of age-related macular degeneration, diabetic macular edema, and diabetic retinopathy (excluding diabetic macular edema), and
a route of administration is intravitreal administration.

8. A method for prophylaxis or therapy of a retinal disease comprising administering to a patient a pharmacologically effective amount of a JNK-inhibitory peptide less than 150 amino acids in length, containing at least one D-amino acid, and having (a) a JNK-inhibitory sequence of at least any of SEQ ID NO: 1 and SEQ ID NO: 2, and (b) a transport sequence of at least any of SEQ ID NO: 3 and SEQ ID NO: 4.

9. A method for prophylaxis or therapy of a retinal disease comprising administering to a patient a pharmacologically effective amount of a JNK-inhibitory peptide less than 150 amino acids in length, containing at least one D-amino acid, and having an amino acid sequence of at least any of SEQ ID NO: 5 and SEQ ID NO: 6.

10. A method for prophylaxis or therapy of a retinal disease comprising administering to a patient a pharmacologically effective amount of a JNK-inhibitory peptide consisting of an amino acid sequence of SEQ ID NO: 5 or SEQ ID NO: 6, and containing at least one D-amino acid.

11. The method for prophylaxis or therapy according to claim 10, wherein
all of the amino acids in said JNK-inhibitory peptide are D-amino acids.

12. The method for prophylaxis or therapy according to any of claims 8 to 11, wherein
said retinal disease is at least one selected from the group consisting of age-related macular degeneration, diabetic macular edema, diabetic retinopathy (excluding diabetic macular edema), central exudative chorioretinopathy, angioid streaks, retinal pigment epithelium detachment, multifocal choroiditis, neovascular maculopathy (limited only to case caused by high myopia, tilted disc syndrome, or choroidal osteoma), retinopathy of prematurity, retinitis pigmentosa, Leber's disease, retinal artery occlusion, retinal vein occlusion, central serous chorioretinopathy, retinal macroaneurysm, retinal detachment, proliferative vitreoretinopathy, Stargardt's disease, choroidal sclerosis, chorioderemia, vitelliform macular dystrophy, Oguchi's disease, fundus albipunctatus, retinitis punctata albescens, and gyrate atrophy of choroid and retina.

13. The method for prophylaxis or therapy according to any of claims 8 to 11, wherein
a route of administration is intravitreal administration, administration into conjunctival sac, subconjunctival administration, or sub-tenon administration.

14. The method for prophylaxis or therapy according to claim 11, wherein
said retinal disease is at least one selected from the group consisting of age-related macular degeneration, diabetic macular edema, and diabetic retinopathy (excluding diabetic macular edema), and

a route of administration is intravitreal administration.

**15.** Use of a JNK-inhibitory peptide for manufacturing a prophylactic or therapeutic agent for a retinal disease, said JNK-inhibitory peptide being less than 150 amino acids in length, containing at least one D-amino acid, and having (a) a JNK-inhibitory sequence of at least any of SEQ ID NO: 1 and SEQ ID NO: 2, and (b) a transport sequence of at least any of SEQ ID NO: 3 and SEQ ID NO: 4.

**16.** Use of a JNK-inhibitory peptide for manufacturing a prophylactic or therapeutic agent for a retinal disease, said JNK-inhibitory peptide being less than 150 amino acids in length, containing at least one D-amino acid, and having an amino acid sequence of at least any of SEQ ID NO: 5 and SEQ ID NO: 6.

**17.** Use of a JNK-inhibitory peptide for manufacturing a prophylactic or therapeutic agent for a retinal disease, said JNK-inhibitory peptide consisting of an amino acid sequence of SEQ ID NO: 5 or SEQ ID NO: 6, and containing at least one D-amino acid.

**18.** The use according to claim 17, wherein all of the amino acids in said JNK-inhibitory peptide are D-amino acids.

**19.** The use according to any of claims 15 to 18, wherein said retinal disease is at least one selected from the group consisting of age-related macular degeneration, diabetic macular edema, diabetic retinopathy (excluding diabetic macular edema), central exudative chorioretinopathy, angioid streaks, retinal pigment epithelium detachment, multifocal choroiditis, neovascular maculopathy (limited only to case caused by high myopia, tilted disc syndrome, or choroidal osteoma), retinopathy of prematurity, retinitis pigmentosa, Leber's disease, retinal artery occlusion, retinal vein occlusion, central serous chorioretinopathy, retinal macroaneurysm, retinal detachment, proliferative vitreoretinopathy, Stargardt's disease, choroidal sclerosis, chorioderemia, vitelliform macular dystrophy, Oguchi's disease, fundus albipunctatus, retinitis punctata albescens, and gyrate atrophy of choroid and retina.

**20.** The use according to any of claims 15 to 18, wherein a route of administration is intravitreal administration, administration into conjunctival sac, subconjunctival administration, or sub-tenon administration.

**21.** The use according to claim 18, wherein said retinal disease is at least one selected from the group consisting of age-related macular degeneration, diabetic macular edema, and diabetic retinopathy (excluding diabetic macular edema), and a route of administration is intravitreal administration.

## INTERNATIONAL SEARCH REPORT

| International application No. |
| --- |
| PCT/JP2010/055208 |

A. CLASSIFICATION OF SUBJECT MATTER
*A61K38/00*(2006.01)i, *A61P27/02*(2006.01)i, *A61P43/00*(2006.01)i, *C07K7/06*(2006.01)n, *C07K7/08*(2006.01)n, *C07K14/00*(2006.01)n, *C12N9/99*(2006.01)n

According to International Patent Classification (IPC) or to both national classification and IPC

B. FIELDS SEARCHED

Minimum documentation searched (classification system followed by classification symbols)
A61K38/00, A61P27/02, A61P43/00, C07K7/06, C07K7/08, C07K14/00, C12N9/99

Documentation searched other than minimum documentation to the extent that such documents are included in the fields searched
| | | | |
| --- | --- | --- | --- |
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CA/REGISTRY/MEDLINE/EMBASE/BIOSIS(STN),
JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
| --- | --- | --- |
| X<br>Y | Roduit R. et al., MAP kinase pathways in UV-induced apoptosis of retinal pigment epithelium ARPE19 cells., Apoptosis, 2008, Vol.13, No.3, pages 343 to 353, ISSN: 1360-8185 | 1-5,15-19<br>6,7,20,21 |
| Y | Chan C.M. et al., Protective effects of (-)-epigallocatechin gallate on UVA-induced damage in ARPE19 cells., Molecular Vision, 2008, Vol.14, pages 2528 to 2534, ISSN: 1090-0535 | 1-7,15-21 |
| Y | Tezel G. et al., Role of tumor necrosis factor receptor-1 in the death of retinal ganglion cells following optic nerve crush injury in mice., Brain Research, 2004, Vol.996, No.2, pages 202 to 212, ISSN: 0006-8993 | 1-7,15-21 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 16 April, 2010 (16.04.10) | 11 May, 2010 (11.05.10) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

**INTERNATIONAL SEARCH REPORT**

| | International application No. |
|---|---|
| | PCT/JP2010/055208 |

C (Continuation).   DOCUMENTS CONSIDERED TO BE RELEVANT

| Category* | Citation of document, with indication, where appropriate, of the relevant passages | Relevant to claim No. |
|---|---|---|
| Y | Hirt L. et al., D-JNKI1, a cell-penetrating c-Jun-N-terminal kinase inhibitor, protects against cell death in severe cerebral ischemia., Stroke, 2004, Vol.35, No.7, pages 1738 to 1743, ISSN: 0039-2499 | 1-7,15-21 |
| Y | Repici M. et al., Specific JNK inhibition by D-JNKI1 protects Purkinje cells from cell death in Lurcher mutant mouse., Cerebellum, 2008, Vol.7, No.4, pages 534 to 538, ISSN: 1473-4222 | 1-7,15-21 |
| Y | Conrad P.W. et al., Intravitreal bevacizumab has initial clinical benefit lasting eight weeks in eyes with neovascular age-related macular degeneration., Clinical ophthalmology, 2008, Vol.2 No.4, pages 727 to 733, ISSN: 1177-5467 | 6,7,20,21 |
| A | Bonny C. et al., Cell-permeable peptide inhibitors of JNK: novel blockers of beta-cell death., Diabetes, 2001, Vol.50, No.1, pages 77 to 82, ISSN: 0012-1797 | 1-7,15-21 |

Form PCT/ISA/210 (continuation of second sheet) (July 2009)

19

**INTERNATIONAL SEARCH REPORT**

International application No.

PCT/JP2010/055208

| Box No. II | Observations where certain claims were found unsearchable (Continuation of item 2 of first sheet) |
| --- | --- |

This international search report has not been established in respect of certain claims under Article 17(2)(a) for the following reasons:

1. ☒ Claims Nos.: 8-14
   because they relate to subject matter not required to be searched by this Authority, namely:
   Claims 8 to 14 include methods for treatment of the human body by therapy and thus relate to a subject matter which this International Searching Authority is not required, under the provisions of Rule 39.1(iv) of the Regulations under the PCT, to search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III | Observations where unity of invention is lacking (Continuation of item 3 of first sheet) |
| --- | --- |

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008518922 A
- JP 2003511071 A
- JP 2009507502 A

**Non-patent literature cited in the description**

- *Invest. Ophthalmol. Vis. Sci.,* 2007, vol. 48 (1), 455-463
- *Invest. Ophthalmol Vis. Sci.,* 2003, vol. 44 (12), 5383-5395
- *Invest. Ophthalmol. Vis. Sci.,* 2003, vol. 44 (12), 5383-5395
- *Invest. Ophthalmol. Vis. Sci.,* 2010, 51
- *Folia Ophthalmologica Japonica,* 1994, vol. 45, 853-856
- *Expert Rev. Ophthalmol.,* 2008, vol. 3 (1), 29-42
- *Nature,* 1984, vol. 311, 575-577

SEQUENCE LISTING

<110> SANTEN PHARMACEUTICAL CO., LTD.

<120> Prophylactic Or Therapeutic Agent For Retinal Disease And Method
For Prophylaxis Or Therapy Of Retinal Disease Using JNK
(C-Jun Amino-Terminal Kinase)-Inhibitory Peptide, And Use Of The
Peptide

<130> S10973EU

<140> PCT/JP2010/055208
<141> 2010-03-25

<150> JP2009-080992
<151> 2009-03-30

<160> 6

<170> PatentIn version 3.5

<210> 1
<211> 19
<212> PRT
<213> Artificial Sequence

<220>
<223> JNK-inhibitory peptide

<400> 1

Arg Pro Lys Arg Pro Thr Thr Leu Asn Leu Phe Pro Gln Val Pro Arg
1               5                   10                  15


Ser Gln Asp


<210> 2
<211> 19
<212> PRT
<213> Artificial Sequence

<220>
<223> JNK-inhibtory peptide

<400> 2

Asp Gln Ser Arg Pro Val Gln Pro Phe Leu Asn Leu Thr Thr Pro Arg
1               5                   10                  15


Lys Pro Arg


<210> 3
<211> 10
<212> PRT
<213> Artificial Sequence

<220>
<223> transport sequence

<400> 3

Gly Arg Lys Lys Arg Arg Gln Arg Arg Arg
1               5                   10

```
<210>  4
<211>  10
<212>  PRT
<213>  Artificial Sequence

<220>
<223>  transport sequence

<400>  4

Arg Arg Arg Gln Arg Arg Lys Lys Arg Gly
1               5                   10


<210>  5
<211>  31
<212>  PRT
<213>  Artificial Sequence

<220>
<223>  JNK-inhibitory sequence

<400>  5

Gly Arg Lys Lys Arg Arg Gln Arg Arg Arg Pro Pro Arg Pro Lys Arg
1               5                   10                  15


Pro Thr Thr Leu Asn Leu Phe Pro Gln Val Pro Arg Ser Gln Asp
            20                  25                  30


<210>  6
<211>  32
<212>  PRT
<213>  Artificial Sequence

<220>
<223>  JNK-inhibitory sequence

<400>  6

Gly Arg Lys Lys Arg Arg Gln Arg Arg Arg Pro Pro Arg Pro Lys Arg
1               5                   10                  15


Pro Thr Thr Leu Asn Leu Phe Pro Gln Val Pro Arg Ser Gln Asp Thr
            20                  25                  30
```